# EUROPEAN PATENT APPLICATION

(11) **EP 3 476 479 A1**
(43) Date of publication of application: **01.05.2019**
(21) Application number: 17306469.2
(22) Date of filing: 25.10.2017
(51) Int. Cl.: B01J 29/03, B01J 29/00, B01J 35/00, B01J 35/10, C07C 5/333, C07C 11/167

(54) **NEW METALLIC CATALYSTS**

(71) Applicant: CENTRE NATIONAL DE LA RECHERCHE SCIENTIFIQUE, 75016 Paris (FR); Université Claude Bernard Lyon 1, 69100 Villeurbanne (FR)
(72) Inventor: ESSAYEM, Nadine, 38540 Saint Just Chaleyssin (FR); RUSSBUELDT, Bernhard, 37671 Hoexter (DE); FABIANO, Demian Patrick, 36420 Ouro Branco-MG (BR); ETERNOT, Marion, 01480 Frans (FR); HOELDERICH, Wolfgang, 67227 Frankenthal (DE)
(74) Representative: Lavoix

(57) **Abstract**

The present invention relates to a compound a metal and a mesostructured support, wherein said mesostructured support comprises pores separated from each other by pore walls; and wherein said metal comprises atoms which are homogeneously and atomically dispersed at the surface and within the pore walls of said mesostructured support. The present invention also relates to a method of preparation of this compound, and its use as catalyst in a catalyzed reaction.

## Description

The present invention relates to the field of solid catalysts and provides a compound comprising a metal and a mesostructured support. The present invention also relates to a method of preparation of said compound, and its use as a catalyst in chemical reactions.

Heterogeneous catalysts are well known and widely used in chemical reactions. In heterogeneous catalysis, the active sites may be defined as the part of the catalyst which interacts with a reactant in a catalyzed chemical reaction. Solid catalysts, and specifically their active sites and their surface, are well characterized.

One of the disadvantages of these solid catalysts lies in the heterogeneity of their surface, which often leads to a low catalytic efficiency when they are involved in chemical reactions. Many researches are carried out in order to increase the efficiency of such solids, notably by increasing the homogeneity of their surface.

Recently, single-atoms catalysts (SACs) have been developed. These solids typically comprise a metal and a support, the atoms of said metal being dispersed on the support. Said support may be a mesostructured support. In SACs, each metal atom which is exposed to the reactant represents an active site. Accordingly, in comparison to usual solid catalysts which may comprise metallic clusters in which several metal atoms are not accessible, the SACs present more active sites. This presents evident advantages such as enhanced catalytic activity and higher selectivity control.

Up to now, to disperse metal atoms on mesostructured supports, different methods of synthesis have been reported. All these methods aim obtaining a high dispersion of the metal and preserving the mesostructuration of the support. These methods of synthesis may be chosen amongst impregnation methods, post grafting synthesis, and one- step sol-gel methods based on the use of alkoxides. For instance, it is known to use peroxo-complexes of tungsten, molybdenum and titanium in order to prepare tungsten, molybdenum or titanium supported on mesostructured silica (Piquemal et al., Microporous and Mesoporous Materials 29 (1999) 291-304; Briot et al., New J. Chem., 2002, 26, 1443-1447; Chen et al., Applied Catalysis A: General 273 (2004) 195-191; and Cheng et al., Catalysis Communications 8 (2007) 1060-1064).

All these solid catalysts described in the prior art present several drawbacks. Indeed, when they are employed in chemical reactions, the metal atoms comprised in these solids may aggregate and form clusters; or the metal atoms may lixiviate, leading to a decrease of the catalytic activity.

Thus, there is a need to provide a stable catalyst, in other words, a catalyst comprising metal atoms which do not aggregate or form clusters when used in chemical reaction. Moreover, there is a need to provide a catalyst which does not lixiviate during chemical reactions.

An objective of the present invention is thus to provide a stable compound comprising metal atoms which do not aggregate or lixiviate.

Another objective of the invention is to provide a method of preparation of this compound.

One objective of the invention is also to provide a stable compound useful as a catalyst in chemical reactions.

Thus, the present invention relates to a compound comprising a metal and a mesostructured support, wherein said mesostructured support comprises pores separated from each other by pore walls; and wherein said metal comprises atoms which are homogeneously and atomically dispersed at the surface and within the pore walls of said mesostructured support.

The compound according to the invention is a solid catalyst which may be implemented in catalyzed reactions involving reactants. Subsequently, the compound may be designated as catalyst.

According to the invention, the expression "mesostructured support" means that the support comprised in the compound has a long-range organization, principally characterized by hexagonal or cubic structures. These structures are characterized by XRD patterns with reflection lines at low 2-θ angles lower than 10° as explained in J.S.Beck et al. from Mobil in J.Am.Chem.Soc. 1992, 114, 27, 10834).

According to the invention, the "dispersion" of metal atoms on a support represents the number of metal atoms exposed to reactants with respect to the total number of metal atoms present on said support. The dispersion of a metal on a support may be expressed as a percentage.

Within the present application, the tem "pores" designs the cavities which may be present on a solid compound. Typically, pores can have regular or irregular shapes and may be partly or fully connected between each other.

Within the present application, the expression "pore walls" designs the part of the compound located between two distinct pores.

Within the present application, the expression "dispersed within the pore walls of the mesostructured support" means that the metal atoms are present in the bulk of the pore walls.

According to the invention, the expression "atomically dispersed" means that the compound only comprises single metal atoms separated by each other. The metal atoms are not aggregated and do not form clusters. In other words, the expression "atomically dispersed" means that all the metal atoms comprised in the compound of the invention are exposed to reactants.

Within the present application, the expression "homogenously dispersed" means that the atomic concentration of the metal atoms measured wherever on the surface and within the pore walls of the mesostructured support equals the average atomic concentration of metal atoms measured on the surface and within the pore walls of the mesostructured support. Within the present application, the "average atomic concentration" represents the average of all the atomic concentrations measured anywhere on the surface and within the pore walls of the mesostructured support. The atomic concentration may represent either the number of metal atoms at the surface of the pore walls of the mesostructured support with respect to the total number of atoms present at the surface of the pore walls of the mesostructured support (superficial atomic concentration) or the number of metal atoms within the pore walls of the mesostructured support with respect to the total number of atoms within the pore walls of the mesostructured support (bulk atomic concentration).

Subsequently, the expression "comprised between X and Y" means a range which includes X and Y.

Preferably, the metal according to the invention is chosen from the group consisting of transition metals, noble metals, and mixtures thereof. Advantageously, the metal is a transition metal, and preferably the metal is an early d-block transition metal. Early d-block transition metals are metals selected from the group consisting of: scandium, yttrium, titanium, zirconium, hafnium, vanadium, niobium, tantalum, chromium, molybdenum, tungsten, manganese, technetium and rhenium.

In a preferred embodiment, the metal is chosen from the group consisting of: tantalum, tungsten, niobium, molybdenum, titanium, and mixtures thereof. Advantageously, the metal is tantalum or tungsten.

In the compound according to the invention, the metal atoms are dispersed on the surface and within the pore walls of the mesostructured support. Theses metal atoms represent the active sites of the compound. Thanks to the fact the metal atoms are dispersed within the pore walls of the mesostructured support, the compound presents an excellent stability. Specifically, the metal atoms present at the surface and within the pore walls do not aggregate or lixiviate. According to the invention, the term "stable" means that the metal atoms do not aggregate or form clusters, and do not lixiviate during their use as a catalyst.

Preferably, in the compound according to the invention, the diameter of the metal atoms is comprised between 1 nm and 1 A, advantageously between 5 A and 1 A. The diameter of the metal atoms is measured by Transmission Electron Microscopy (TEM) and High-Angle Annular Dark-Field Scanning Transmission Electron Microscopy (HAADF-STEM). HAADF-STEM is a STEM method which receives inelastically scattered electrons or thermal diffuse scattering at high angles using an annular dark-field detector.

In a preferred embodiment, the superficial atomic concentration of the metal atoms is comprised between 0.1% and 5%. The superficial atomic concentration of the metal atoms is measured by X-ray photoelectron spectroscopy (XPS).

In a preferred embodiment, the metal dispersion is higher than 99%, preferably the metal dispersion equals 100%. Metal dispersion is deduced from Transmission Electron Microscopy (TEM) analysis.

The mesostructured support according to the invention may comprise an element chosen from the group consisting of: metalloids, post-transition metals, transition metals and mixtures thereof. Advantageously, the mesostructured support comprises an element chosen from the group consisting of: silicon, zirconium, aluminum and titanium.

According to a preferred embodiment, the mesostructured support is an oxide based mesostructured support. Advantageously, the mesostructured support is chosen from the group consisting of supports made of mesostructured silica, mesostructured zirconia, mesostructured alumina, and mesostructured titanium dioxide. Preferably, the support is mesostructured silica.

Advantageously, the support is chosen from the group consisting of: MCM-41, KIT-6 and SBA-15. Preferably, the support is MCM-41 or SBA-15, and more preferably, the support is MCM-41.

MCM-41, SBA-15 and KIT-6 are mesoporous silica materials presenting different pore geometries, pore wall thicknesses and pore diameters.

Preferably, the mesostructured support is mesoporous, meaning that the pores of the support are mesopores, *i.e.* the diameter of the pores is comprised between 2 nm and 50 nm, preferably between 3 nm and 15 nm.

Advantageously, the thickness of the pore walls of the mesostructured support is comprised between 0.5 nm to 30 nm, preferably between 0.5 nm and 10 nm.

Particularly, the specific surface area of the mesostructured support according to the invention is comprised between 400 m²/g and 1,500 m²/g, more preferably between 600 m²/g and 1,400 m²/g.

The specific surface area, or BET (Brunauer, Emmet and Teller) surface area, the pore size of the compound according to the invention are measured by N₂ adsorption-desorption isotherms, measured at 77 °K or (-196.15 °C) after degassing off said compound at 0.0001 bar. The pore wall thickness is measured by comparing the results obtained from XRD (X-ray diffraction) analysis and the results obtained from N₂ adsorption-desorption isotherms.

Advantageously, in the compound according to the invention, the atomic ratio between the element of the support and the dispersed metal atoms is comprised between 20 and 400, preferably between 50 and 100.

In a preferred embodiment, the atomic ratio between the element of the support and the dispersed metal atoms measured at the surface of the pore walls is equal to the atomic ratio between the element of the support and the dispersed metal atoms measured within the pore walls. This specific feature implies that the metal atoms do not aggregate, and are thus dispersed everywhere in the surface and within the pore walls of the support.

The atomic ratio between the element of the support and the dispersed metal atoms within the pore walls is deduced from the chemical analysis of the element of support (Si, Zr, Al, Ti, etc.) and the chemical analysis of the metal. The atomic ratio between the element of the support and the dispersed metal atoms measured at the surface of the pore walls are measured by X-ray photoelectron spectroscopy (XPS).

Advantageously, the compound according to the invention is doped with a metallic co-catalyst. Said metallic co-catalyst may be supported on silica. Preferably, the metallic co-catalyst is copper supported on silica or silver supported on silica. The compound according to the invention may be doped with a metallic co-catalyst by mechanical mixture, or by impregnation. Preferably, the amount of metal of the metallic co-catalyst comprised in the compound of the invention is comprised between 5% and 20% by weight with respect to the total weight of the compound. Preferably, the amount of metal of the metallic co-catalyst comprised in the compound of the invention equals to 10% by weight with respect to the total weight of the compound.

The present invention also relates to a process of preparation of the compound according to the invention, said process comprising the following steps:
i) solubilization of a metal precursor in an aqueous hydrogen peroxide solution at a temperature comprised between 0°C and 200°C, and obtaining a solution A of a metallic peroxo-complex;
ii) mixing in water:
   a. a source of the support; and
   b. at least a template;
   for obtaining a suspension B;
iii) addition of solution A obtained at step i) into suspension B obtained at step ii), and obtaining a solution C;
iv) aging of solution C obtained at step iii), and obtaining an aged solution;
v) hydrothermal synthesis of the aged solution obtained at step iv), and obtaining a solution D;
vi) filtration of solution D obtained at step v), and obtaining a filtered solid;
vii) washing of the filtered solid obtained at step vi), and obtaining a washed and filtered solid;
viii) calcination of the washed and filtered solid obtained at step vi), and obtaining a compound comprising metal atoms homogeneously and atomically dispersed on a mesostructured support.

Advantageously, the process according to the invention is a one-pot process. In said process, the metallic peroxo-complex is added into suspension B, and is thus present during the synthesis of the mesostructured support. Thus, the metal atoms are dispersed on the surface and within the pore walls of the mesostructured support during the support synthesis.

Preferably, during step i), the aqueous H₂O₂ solution comprises tetramethyl ammonium hydroxide (TMAOH) or tetraethyl ammonium hydroxide (TEAOH) as hydroxide source. The pH of the solution is about 13. The presence of these compounds enables the isolation of the metal element as monomeric species, and thus the formation of metallic clusters of the metal atoms is avoided.

In a preferred embodiment, the metal precursor is a hydrated oxide-hydroxide of the metal, represented by the formula (I):

MOₓ(OH)_{y}(OH)₂ (I)

wherein M represents the metal, x and y are comprised between 0 and 7.

The metal precursor may be obtained by precipitation of a solution of a metal halide in an ammonia solution, which may comprise HBr. The metal precursor is advantageously prepared at the beginning of step i).

Specifically, the metal precursor, which is totally soluble in aqueous H₂O₂ solution, is added directly under vigorous stirring to the aqueous H₂O₂ solution or after its dissolution in HBr when HBr is used.

In solution A, the metallic peroxo-complexes are isolated, which means that they do not form cluster or polymeric species containing several metallic atoms. Detailed definitions of peroxo-complexes may be found in "D.Bayoy, M.Devillers Coordination Chemistry Reviews 250 (2006) 2610-2626 or in "V.S.Sergienko Crystallography Reports 53(1), 2008, 18-46".

In a preferred embodiment, step i) is carried out at a temperature comprised between 0°C and 180°C. Preferably, step i) is carried out at a temperature comprised between 0°C and 20°C, more preferably between 0°C and 10°C, and advantageously, the temperature of step i) equals 0°C.

In step ii), the source of the support is an inorganic or organic compound. When the support is mesostructured silica, the silicon source may be chosen amongst amorphous silica, silicates, silicon alkoxide, and mixtures thereof.

The template is also named organic directing agent or templating agent. This template may be ionic or neutral. When the template is ionic, it may be chosen amongst quaternary ammonium salts such as cetyltrimethylammonium bromide or cetyltrimethylammonium chloride. An ionic template may be used to obtain a support such as MCM-41. When the template is neutral, it may be chosen amongst amphiphilic macromolecules such as triblock copolymer, for example Pluronic P123 (EO₂₀PO₇₀EO₂₀). A neutral template may be used to obtain a support such as SBA-15. In step ii), suspension B may also comprise a surfactant. Typically, step ii) is carried out at a temperature comprised between 20°C and 80°C, preferably at a temperature of 60°C.

Step iii) may be carried out under stirring in an autoclave. Preferably, when an autoclave is used, it is closed immediately after the addition of solution A. In a preferred embodiment, solution C has the consistency of a "gel". Particularly, step iii) is carried out at a temperature comprised between 20°C and 50°C, preferably step iii) is carried out at 35°C.

Step iv) may comprise two aging steps. The first aging step is preferably carried out during 0 to 48 hours, preferably during 24 hours, and at a temperature comprised between 20°C and 50°C, preferably at a temperature equal to 35°C. The second aging step is advantageously carried out during 0 to 48 hours, preferably during 24 hours, and at a temperature comprised between 50°C and 95°C, preferably at a temperature of 90°C.

Step v) may be carried out at a temperature comprised between 100°C and 180°C, preferably at 150°C. Preferably, step v) is carried out during 48 hours.

Depending on the source of the support and of the template, different mesostructured supports may be prepared. Steps iv) and v) represent the synthesis of the mesostructured support of the compound according to the invention.

Advantageously, step vii) is carried out with water until the pH of the washing water equals 9. After step vii), the solid may be dried under ambient air during 24 hours.

Typically, step viii) is carried out under air at a temperature higher than 450°C and during more than 2 hours. Preferably, step viii) is carried out at 550°C for 6h.

Preferably, the compound obtained with the process of the invention is not polluted with elements present such as salts, which may be present when the compound is prepared with the processes described in prior art.

In a preferred embodiment, the process of the invention comprises a step ix) carried out after the step viii) in which the compound obtained at the end of step viii) is mechanically admixed with a metallic co-catalyst, said metallic co-catalyst being copper supported on silica or silver supported on silica.

Preferably, in step ix), the amount of metal of the co-catalyst is comprised between 5% and 20%, and preferably 10% by weight with respect to the total weight of the compound.

The present invention also relates to the use of the compound according to the invention as a catalyst in a catalyzed reaction. Advantageously, the reaction is an acid-catalyzed reaction. In a preferred embodiment, the catalyzed reaction is chosen from the group consisting of oxidation reaction, alcohol dehydration, and olefins metathesis.

Preferably, the catalyzed reaction is chosen from the group consisting of butadiene synthesis from ethanol dehydration, and oxidative cleavage of vegetable oils with oxygen peroxide.

When the compound of the invention is used in the butadiene synthesis, the metal comprised in said compound is preferably tantalum or tungsten. When the reaction is oxidative cleavage of vegetable oils, the metal comprised in said compound is preferably tantalum or titanium.

When the compound of the invention is used for butadiene synthesis from ethanol, said compound may be doped with copper.

When the compound according to the invention is used for the butadiene synthesis and the starting reactive medium is an equimolar mixture of ethanol with acetaldehyde, the compound does not need to be doped.

### FIGURES

Figure 1 represents the low angles patterns of MCM-41 synthesized according to example 1 and of compounds synthesized according to examples 2-4 and 6.
Figure 2 represents the low angles patterns of MCM-41 synthesized according to example 1 and of compounds synthesized according to examples 5-8.
Figure 3 represents the N₂ isotherm adsorption over MCM-41 synthesized according to example 1 and the compound synthesized according to example 6.
Figure 4 represents the N₂ isotherm adsorption over MCM-41 synthesized according to example 1 and the compound synthesized according to example 5.
Figure 5 represents the typical structuration of MCM-41 synthesized according to example 1 analyzed by TEM
Figure 6 represents the typical structuration of the compound synthesized according to example 6 and analyzed by TEM
Figure 7 represents the typical structuration of the compound synthesized according to example 5 and analyzed by TEM
Figures 8-11 represent different pictures of the typical structuration of the compound synthesized according to example 5 and analyzed by STEM-HAADF.

The different aspects of the invention are illustrated by the following examples, in which the Si/M ratio represents an atomic ratio where M is a metal.

### Example 1: Preparation of MCM-41

164.01 g of cetyltrimethylammonium bromide (CTMABr) surfactant, obtained from Fluka™ or Aldrich®, is dissolved at 35°C in 896.83 g of water. To this mixture, 293.45 g of an aqueous solution of tetramethyl ammonium silicate (TMA₁₅SiO₂, 11.7% SiO₂) is added in 5 min with a dropping funnel. The aqueous solution of TMA_{1.5}SiO₂, 11.7%SiO₂ is obtained by mixing 150.20 g of an aqueous solution of TMA_{0·5}SiO₂ (40% SiO₂), obtained from SACHEM © with 182.30 g of an aqueous solution of tetramethylammonium hydroxide (25%) (TMAOH) obtained from Aldrich®. The mixture is heated up in a PET bottle for 3 hours at 90°C in a water bath to achieve the complete depolymerization of TMA_{0·5}SiO₂. The aqueous solution of TMA_{1.5}SiO₂, 11.7%SiO₂ is heated up to approximately 60°C before use to obtain a clear solution. After, 364.99 g Ludox® AS40 40% SiO₂ colloid solution, provided by Aldrich® is added in 5 min. The white suspension is transferred into 2.4 L stirred autoclave pre-heated at 35°C. Aging is done for 24 h at 35°C followed by 24 h at 90°C. Finally, hydrothermal synthesis is performed for 48 h at 150°C and 4 bars under stirring 180 rpm. After cooling to 50°C, the white powder is separated by filtration, washed with water and dried at room temperature. The solid is calcined in air at 550°C for 360 min (1°C/min).

### Example 2: Preparation of Ti-MCM-41, Si/Ti = 100

### Preparation of Ti peroxo complex solution:

TiBr₄ solution (0.8663 mol/kg) is prepared starting from TiCl₄ solution, then precipitation of TiO₂.xH₂O with NH₃ solution and dissolution in HBr. The Ti peroxo complex is prepared by cooling a mixture of 8.75 g of H₂O₂ 70%, 65.63 g of tetramethyl ammonium hydroxide (TMAOH) 25% solution and 10 g of water in an ice bath. Under vigorous stirring and cooling, 34.63 g TiBr₄ solution is added. The Ti peroxo complex is forming an intense yellow solution, which is finally diluted with water to 129.23 g in total.

### Hydrothermal synthesis of Ti-MCM-41

A cetyltrimethylammonium hydroxide (CTMAOH) 7.89 % solution is prepared by anionic exchange from 750 mL of Amberlyst-26 OH obtained from Aldrich®, corresponding approximatively to 0.75 mol ion exchange capacity, and 640g of a cetyltrimethylammonium chloride 25% (CTMACI) aqueous solution diluted with water. 120.37 g CTMABr surfactant is dissolved at 35°C in 401.72 g of water and 458.63 g of cetyltrimethylammonium hydroxide (CTMAOH) 7.89% solution. To this mixture, 231.67 g TMA_{1.5}SiO₂ solution is added in 5 min with a dropping funnel and vigorous stirring. TMA_{1.5}SiO₂ solution must be heated up to approximately 60°C before use to obtain a solution again. After, 383.01 g Ludox AS40 SiO₂ colloid is added in 5 min. The white suspension is transferred into 2.4 L stirred autoclave pre-heated at 35°C. The Ti peroxo complex solution is added all at once while stirring at 180 rpm. The autoclave is closed immediately and the gel is aged for 24 h at 35°C followed by 24 h at 90°C. The autoclave is opened to vent the formed O₂ pressure. Hydrothermal synthesis is performed for 48 h at 150°C and 4 bars under stirring. After cooling to 50°C, the white powder is separated by filtration, washed with water until the washing water has a pH equal to 9 and dried at room temperature. The solid is calcined in air at 550°C for 360 min (1°C/min).

The theoretical Ti proportion is equal to 1.31 wt%. The experimental Ti proportion measured by chemical analysis is equal to 1.44 wt%

### Example 3: Preparation of Zr-MCM-41, Si/Zr = 100

### Preparation of Zr peroxo complex solution:

The Zr peroxo complex is prepared by cooling a mixture of 8.75 g of H₂O₂ 70% and 43.75 g of TMAOH 25% solution in an ice bath under vigorous stirring and cooling. 36.04 g ZrOBr₂ solution 0.8325 mol/kg) is added. The Zr peroxo complex is forming an almost colorless solution, which is finally diluted with water to 121.29 g in total.

### Hydrothermal synthesis of Zr-MCM-41

142.14 g CTMABr surfactant is dissolved at 35°C in 603.84 g of water and 229.32 g of CTMAOH 7.89% solution. To this surfactant mixture, 252.26 g TMA_{1.5}SiO₂ solution is added in 5 min with a dropping funnel and vigorous stirring. After, 377.00 g Ludox AS40 SiO₂ colloid is added in 5 min. The white suspension is transferred into 2.4 L stirred autoclave pre-heated at 35°C. The Zr peroxo complex solution is added all at once while stirring at 180 rpm. The autoclave is closed immediately and the gel is aged for 24 h at 35°C followed by 24 h at 90°C. The autoclave is opened to vent the formed O₂ pressure. Hydrothermal synthesis is performed for 48 h at 150°C and 4 bars under stirring. After cooling to 50°C, the white powder is separated by filtration, washed with water until the washing water has a pH equal to 9 and dried at room temperature. The solid is calcined in air at 550°C for 360 min (1 °C/min).

The theoretical Zr proportion is equal to 2.01 wt%. The experimental Zr proportion measured by chemical analysis is equal to 2.06 wt%.

### Example 4: Preparation of Nb-MCM-41, Si/Nb = 100

### Preparation of Nb peroxo complex solution:

18.75 g of H[NbF₆] solution (1.6004 mol/kg) are diluted to 100 mL in a PTFE beaker and after addition of 30.45 mL NH₃ 25% diluted to 100 mL, white Nb₂O₅.xH₂O is precipitated. The fine powder is separated by filtration and washed with 500 mL water. The wet filter cake is transformed into a slurry with 43.75 g TMAOH 25% solution and additional water. While cooling with ice, the Nb₂O₅.xH₂O is dissolved by addition of 11.67 g H₂O₂ 70% solution. The Nb peroxo complex is forming a light yellow solution. The solution is filled up with water to 117.77 g in total.

### Hydrothermal synthesis of Nb-MCM-41

164.01 g CTMABr surfactant is dissolved at 35°C in 815.06g of water. To this surfactant solution, 252.26g TMA_{1.5}SiO₂ solution is added in 5 min with a dropping funnel and vigorous stirring. After, 377.00 g Ludox AS40 SiO₂ colloid is added in 5 min. The white suspension is transferred into 2.4L stirred autoclave pre-heated at 35°C. The Nb peroxo complex solution is added all at once while stirring at 180rpm. The autoclave is closed immediately and the gel is aged for 24 h at 35°C followed by 24 h at 90°C. The autoclave is opened to vent the formed O₂ pressure. Hydrothermal synthesis is performed for 48 h at 150°C and 4 bars under stirring. After cooling to 50°C, the white powder is separated by filtration, washed with water until the washing water has a pH equal to 9 and dried at room temperature. The solid is calcined in air at 550°C for 360 min (1°C/min).

The theoretical Nb proportion is equal to 2.16 wt%. The experimental Nb proportion measured by chemical analysis is equal to 2.35 wt%.

### Example 5: Preparation of Ta-MCM-41, Si/Ta = 50:

### Preparation of Ta peroxo complex solution:

84.91 g of H₂[TaF₇] solution (0.7066 mol/kg) are diluted with water to 250 mL in a PTFE beaker, fresh Ta₂O₅.xH₂O is precipitated by addition of 71.06 mL NH₃ 25% and diluted with 250 mL of water. The solid is separated by filtration, washed with water, and dissolved in an ice cold mixture of 87.5 g of TMAOH 25% and 23.34 g of H₂O₂ 70% and some additional water. A colorless solution is obtained. A total weight of 186.57 g Ta reagent solution is obtained.

### Hydrothermal synthesis of Ta-MCM-41

Similar procedure than for the hydrothermal synthesis of example 4 is applied with 837.5g of water.

The theoretical Ta proportion is equal to 6.85 wt%. The experimental Ta proportion measured by chemical analysis is equal to 7.32 wt%.

### Example 6: Preparation of Ta-MCM-41, Si/Ta = 100

### Preparation of Ta peroxo complex solution:

Similar procedure to the procedure of example 5 is followed using the following amounts of reactants: 42.46 g H₂[TaF₇], 35.5 mL NH₃, 43.7 g TMAOH and 11.67 g H₂O₂. A total weight of 98.97 g Ta reagent solution is obtained.

### Hydrothermal synthesis of Ta-MCM-41

Similar procedure than for the hydrothermal synthesis of example 4 is applied.

The theoretical Ta proportion is equal to 3.55 wt%. The experimental Ta proportion measured by chemical analysis is equal to 3.70 wt%.

### Example 7: Preparation of Ta-MCM-41, Si/Ta = 200

### Preparation of Ta peroxo complex solution:

Similar procedure to the procedure of example 5 is applied using the following amounts of reactants: 21.23 g H₂[TaF₇], 17.76 mL NH₃, 21.88 g TMAOH and 5.84 g H₂O₂. A total weight of 75.2 g Ta reagent solution is obtained.

### Hydrothermal synthesis of Ta-MCM-41

Similar procedure than for the hydrothermal synthesis of example 4 is applied.

The theoretical Ta proportion is equal to 1.81 wt%. The experimental Ta proportion measured by chemical analysis is equal to 1.91 wt%.

### Example 8: Preparation of Ta-MCM-41, Si/Ta = 400

### Preparation of Ta peroxo complex solution:

Similar procedure to the procedure of example 5 is applied using the following amounts of reactants: 10.61 g H₂[TaF₇], 8.88 mL NH₃, 10.94 g TMAOH and 2.92 g H₂O₂. A total weight of 43.12 g Ta reagent solution is obtained.

### Hydrothermal synthesis of Ta-MCM-41

Similar procedure than for the hydrothermal synthesis of example 4 is applied.

The theoretical Ta proportion is equal to 0.91 wt%. The experimental Ta proportion measured by chemical analysis is equal to 1.04 wt%.

### Example 9: X-ray diffraction analyses

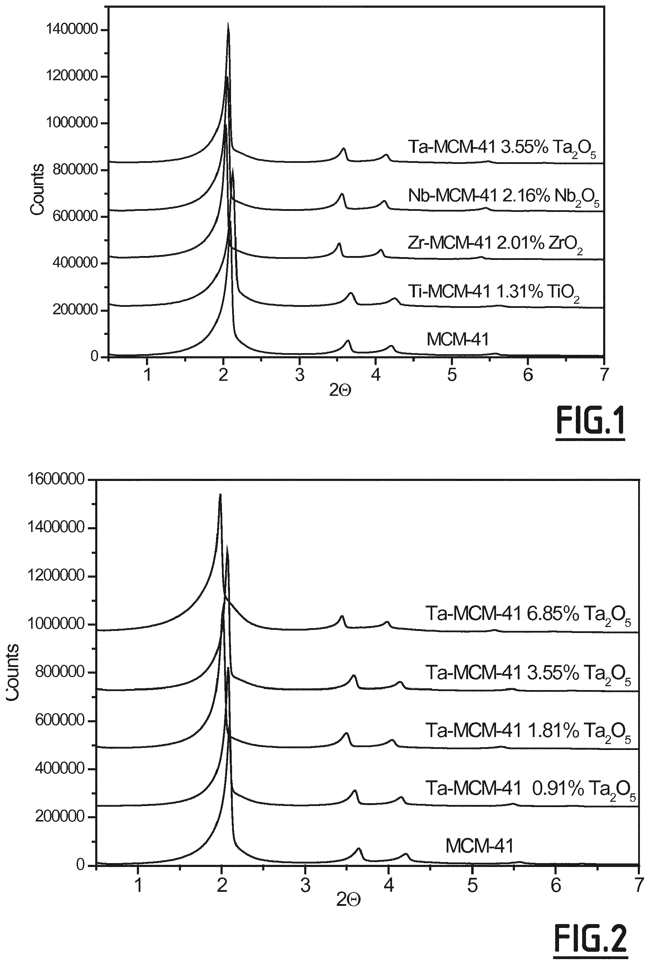

Figures 1 and 2 show the low angles X-ray diffraction of the different compounds synthesized in examples 1-8.

X-ray diffraction characterizes the support periodicity at the nanometric scale and therefore the mesostructuration of the support. The nature of the transition metal (Ti, Zr, Nb or Ta) has a small influence on the position of the 2-θ angle of the main peak which characterizes the periodicity (figure 1). Insertion of the smallest transition elements (titanium) slightly shifts the peak angle position towards higher angles, indicating a small decrease of the pores diameter. By contrast, insertion of largest elements (zirconum, nobium, tantalum) leads to a small shift of the diffraction peak towards lower angles, showing enlarged reticular distances leading to slight increased pore diameters.

Increased amounts of the largest earlier transition metal (tantalum) up to Si/Ta atomic ratio equal to 50, induces a linear decrease of the 2-θ angle position together with the broadening of the XRD diffraction peak (figure 2). This indicates a small enlarging of the reticular distance, and therefore a small increase of the pores diameters, but this indicates also that insertion of high amounts of large transition metal element such as Ta, up to Si/Ta atomic ratio of 50, remains compatible with the mesostructuration of the silica support.

### Example 10: N₂ adsorption isotherms

The textural features of Ta-MCM41 compounds synthesized according to examples 1 and 5-8 are reported in Table 1. It is shown that high BET surface areas are measured whatever the amount of Ta: the surface areas are comprised between 950 m²/g and 1080 m²/g.

**Table 1: Textural characteristic deduced from N₂ adsorption isotherms.**

| Compound synthesized according to | Ta₂O₅ (wt%) | BET area (m².g⁻¹) | Micro area (m².g⁻¹) |
|---|---|---|---|
| Example 1 | - | 1079 | 0 |
| Example 8 | 0.91 | 1017 | - |
| Example 7 | 1.81 | 976 | - |
| Example 6 | 3.5 | 1006 | 0 |
| Example 5 | 6.85 | 952 | 0 |

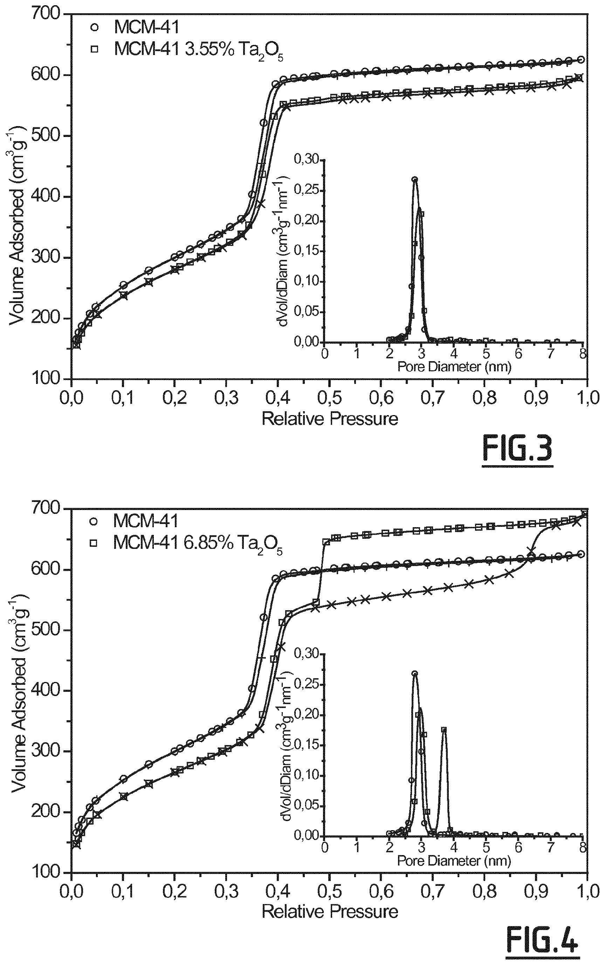

N₂ isotherms of MCM-41 and Ta-MCM-41 with a Si/Ta atomic ratio of 50 and 100 show that upon Ta insertion, the mesostructuration of the silica support is still observed (Figure 3 and Figure 4). The material prepared with a Si/Ta atomic ratio of 100 exhibits regular pores with pore diameters equivalent to that of metal free MCM-41, with pore diameters of 3 nm (Figure 3). For the highest Ta amount (Si/Ta atomic ratio of 50), N₂ isotherm shows a hysteresis at higher partial pressure (Figure 4) corresponding to the presence of larger pores with pores diameters of 3.7 nm in addition to the initial regular pores system characterized by pore apertures of 3 nm. This suggests that the insertion of the transition metal occurs within the silica framework.

### Example 11: Transmission Electron Microscopy (TEM)

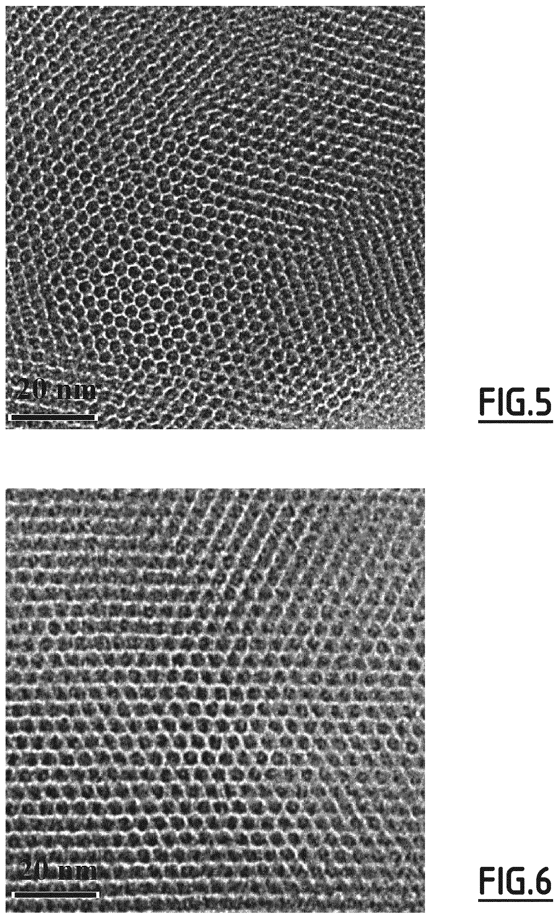

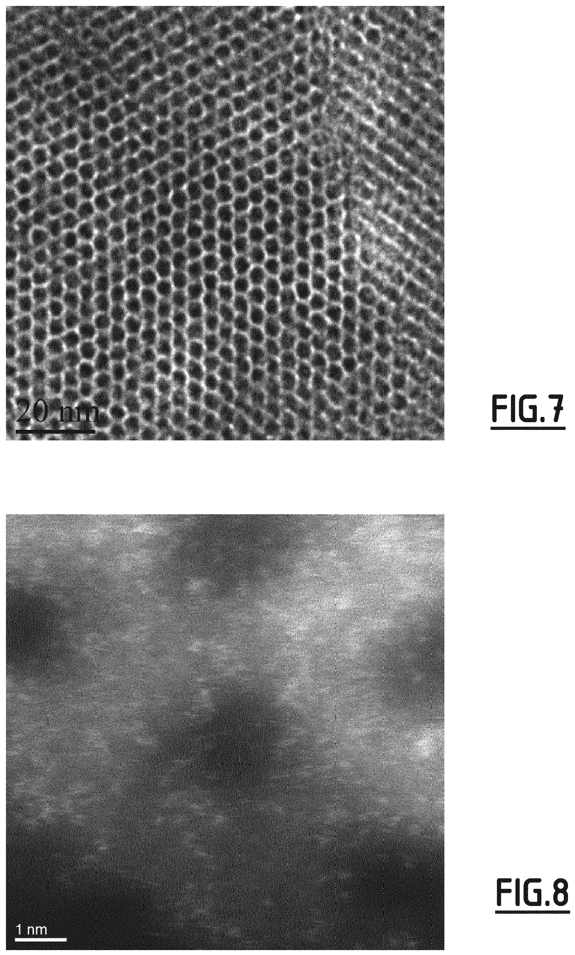

Figure 5 shows the typical structuration of MCM-41 synthesized according to example 1, and Figures 6 and 7 respectively show the typical structuration of the compounds synthesized according to example 6 and 5 observed by TEM.

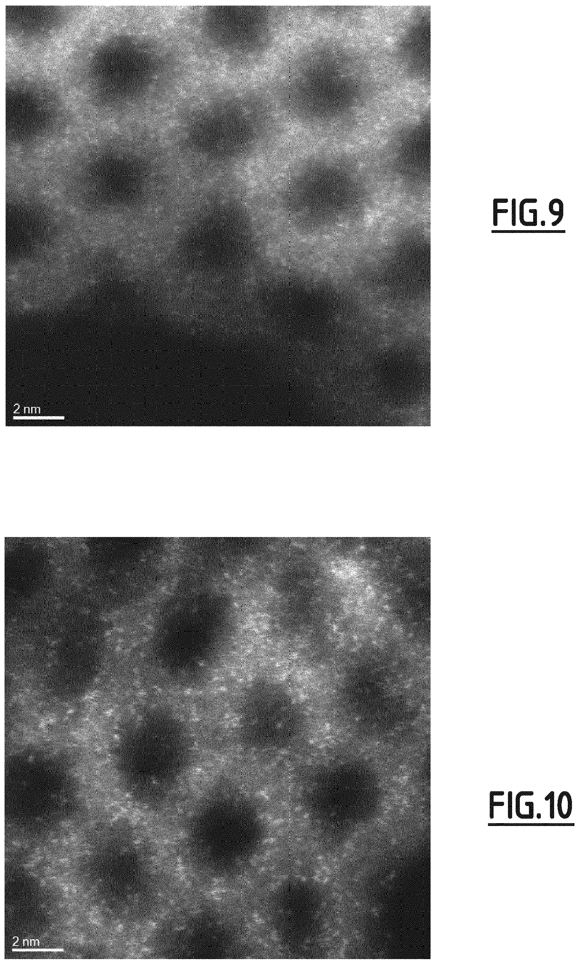

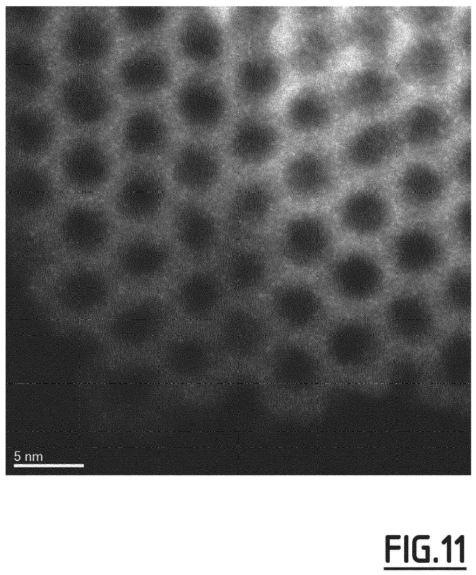

Figures 8-11 show the typical structuration of the compound synthesized according to example 5 and analyzed by STEM-HAADF.

By TEM, the typical structuration of MCM-41 with hexagonal array are observed on Ta-MCM-41 prepared with a Si/Ta atomic ratio of 100 (3.55% Ta₂O₅) or 50 (6.85% Ta₂O₅).

STEM-HAADF of Ta-MCM-41 prepared with the highest amount of Ta (Si/Ta=50) shows the remarkable homogeneous and extreme dispersion of Ta element at the atomic scale (Figures 8-11).

### Example 12: Bulk and superficial compositions

The bulk and superficial compositions of the catalysts prepared according to examples 2, 3, 4 and 6 are reported in table 2.

Bulk compositions are measured by chemical analysis and superficial compositions are measured by XPS analysis.

**Table 2: Bulk and superficial compositions**

| Compound synthesized according to example | Bulk composition | | Superficial composition | |
|---|---|---|---|---|
| | % atomic M | % atomic Si | % atomic M | % atomic Si |
| 2 (Si/Ti=100) | 1.02 | 98.9 | 1.58 | 98.4 |
| 3 (Si/Zr=100) | 0.92 | 99.1 | 0.59 | 99.4 |
| 4 (Si/Nb=100) | 0.97 | 99.0 | 0.83 | 99.2 |
| 6 (Si/Ta=100) | 0.96 | 99.0 | 0.76 | 99.2 |

The bulk composition is similar to the superficial composition of the different compounds.

### Example 13 (comparative): Butadiene synthesis with Ta/Si catalyst non-atomically dispersed

The catalytic tests were carried out in a continuous flow reactor with a catalyst fixed bed (100 mg). A stream of ethanol vapor (13 Torr), carried by N₂ from a saturator (0 °C), is passed through the catalyst bed. The gaseous reaction products were analyzed online with a gas chromatography equipped with a column CP-PoraBOND Q obtained from Agilent®. The catalyst is synthesized by successive wetness impregnations of tantalum (6.8%) and Cu (10%) on commercial mesoporous silica gel. Copper allows the dehydration of ethanol into acetaldehyde which is itself transformed into butadiene over the Ta elements of the catalyst.

The material is dried at 120ºC for 24 h and calcined at 550ºC for 5h (T program 1ºC.min⁻¹). The catalyst is named Cu(10%)/Ta(6.8%)/SiO₂. Results are presented in the Table 3 below:

**Table 3: products distribution over Cu(10%)/Ta(6.8%)/SiO₂ prepared by conventional wetness impregnation as a function of the reaction temperature (100 mg of catalyst, 1,8L.h⁻¹ total flow rate of ethanol (13 torr) in N₂).**

| Temperature | Products yields (mol%) | | | |
|---|---|---|---|---|
| | ethylene | acetaldehyde | 1,3-butadiene | others |
| 300°C | 18 | 34 | 17 | 4 |
| 350°C | 47 | 14 | 4 | 2 |
| 400°C | 72 | 9 | 5 | 2 |
| 450°C | 92 | 5 | 2 | 1 |

The catalytic results show that the butadiene yields remain below 20% in a large range of temperature, from 300 to 450°C.

### Example 14 (according to the invention): Butadiene synthesis with Ta/MCM-41 compound of the invention

The reaction conditions are similar to those of example 13. The catalyst Cu(10%)/Ta(6.8%)/MCM41 is prepared by incipient wetness impregnation of the Ta(6.8%)/MCM41 compound prepared following the example 5 and using Cu(NO₃)2.5H₂O precursor.

The results dare presented in table 4.

**Table 4: Products yields over Cu(10%)/Ta(6.8%)/MCM41 (200mg catalyst, 0.4L.h⁻¹ total flow rate of ethanol (13 torr) in N₂)**

| Temperature | Products yields (mol %) | | | |
|---|---|---|---|---|
| | ethylene | acetaldehyde | 1,3 butadiene | others |
| 250°C | 3 | 23 | 28 | 14 |
| 300°C | 13 | 8 | 65 | 12 |
| 350°C | 53 | 3 | 36 | 8 |

Results show that butadiene yields higher than 20% can be obtained over a large range of temperature between 250°C and 350°C when a catalytic system based on copper dispersed on Ta atomically dispersed in mesostructured silica is used. The higher butadiene yield, greater than 60%, is obtained at 300°C.

## Claims

1. A compound comprising a metal and a mesostructured support, wherein said mesostructured support comprises pores separated from each other by pore walls; and wherein said metal comprises atoms which are homogeneously and atomically dispersed at the surface and within the pore walls of said mesostructured support.

2. The compound according to claim 1, wherein the mesostructured support comprises an element chosen from the group consisting of: metalloids, post-transition metals, transition metals and mixtures thereof.

3. The compound according to claim 1 or 2, wherein the metal is chosen from the group consisting of transition metals, noble metals, and mixtures thereof.

4. The compound according to any of the preceding claims, wherein the metal is chosen from the group consisting of: tantalum, tungsten, niobium, molybdenum, titanium, and mixtures thereof.

5. The compound according to any of the preceding claims, wherein the diameter of the metal atoms is comprised between 1 nm and 1 A.

6. The compound according to any of the preceding claims, wherein the specific surface area of the mesostructured support is comprised between 400 m²/g and 1,500 m²/g.

7. The compound according to any of the preceding claims, wherein the support is chosen from the group consisting of supports made of mesostructured silica, mesostructured zirconia, mesostructured alumina, and mesostructured titanium dioxide.

8. The compound according to any of the preceding claims, wherein the support is chosen from the group consisting of MCM-41, KIT-6 and SBA-15.

9. The compound according to any of claims 2 to 8, wherein the atomic ratio between the element of the support and the dispersed metal atoms is comprised between 20 and 400, preferably between 50 and 100.

10. The compound according to any of claims 2 to 9, wherein the atomic ratio between the element of the support and the dispersed metal atoms measured at the surface of the pore walls is equal to the atomic ratio between the element of the support and the dispersed metal atoms measured within the pore walls.

11. A process of preparation of the compound according to any of the preceding claims comprising the following steps:
i) solubilization of a metal atom precursor in an aqueous hydrogen peroxide solution at a temperature comprised between 0°C and 200°C, and obtaining a solution A of a metallic peroxo-complex;
ii) mixing in water:
a. a source of the support; and
b. at least a template;
for obtaining a suspension B;
iii) addition of solution A obtained at step i) into suspension B obtained at step ii), and obtaining a solution C;
iv) aging of solution C obtained at step iii), and obtaining an aged solution;
v) hydrothermal synthesis of the aged solution obtained at step iv), and obtaining a solution D;
vi) filtration of solution D obtained at step v), and obtaining a filtered solid;
vii) washing of the filtered solid obtained at step vi), and obtaining a washed and filtered solid;
viii) calcination of the washed and filtered solid obtained at step vi), and obtaining a compound comprising metal atoms homogeneously and atomically dispersed on a mesostructured support.

12. The use of the compound according to any of claims 1 to 10 as a catalyst in a catalyzed reaction.

13. The use according to claim 12, wherein the catalyzed reaction is chosen from the group consisting of oxidation reaction, alcohol dehydration, and olefins metathesis.

14. The use according to claim 12 or 13, wherein the catalyzed reaction is chosen from the group consisting of butadiene synthesis from ethanol dehydration, and oxidative cleavage of vegetable oils with oxygen peroxide.

15. The use according to any of claims 12 to 14, wherein the catalyzed reaction is butadiene synthesis from ethanol, and wherein the compound is doped with copper.
